# EUROPEAN PATENT APPLICATION

(11) **EP 2 241 627 A1**
(43) Date of publication of application: **20.10.2010**
(21) Application number: 09158118.1
(22) Date of filing: 17.04.2009
(51) Int. Cl.: C12N 15/85, C12N 15/90

(54) **Mammalian expression vector**

(71) Applicant: LEK Pharmaceuticals d.d., 1526 Ljubljana (SI)
(72) Inventor: Francky, Andrej, 1234 Menges (SI); Gaser, Dominik, 1234 Menges (SI)
(74) Representative: Leger, Hubert

(57) **Abstract**

The present invention describes new mammalian expression vectors comprising a novel combination of regulatory elements and one or more selection marker gene(s). The vector allows for incorporation of at least one, preferably two or more genes of interest, its/their subsequent expression, and for selection of transfected cells using, e.g., G418 and/or MTX. The pDGPΔGOI vector as an example for a mammalian expression vector according to the present invention exhibits a 9555 bp sequence, one strand of which is represented by SEQ ID NO:2.

## Description

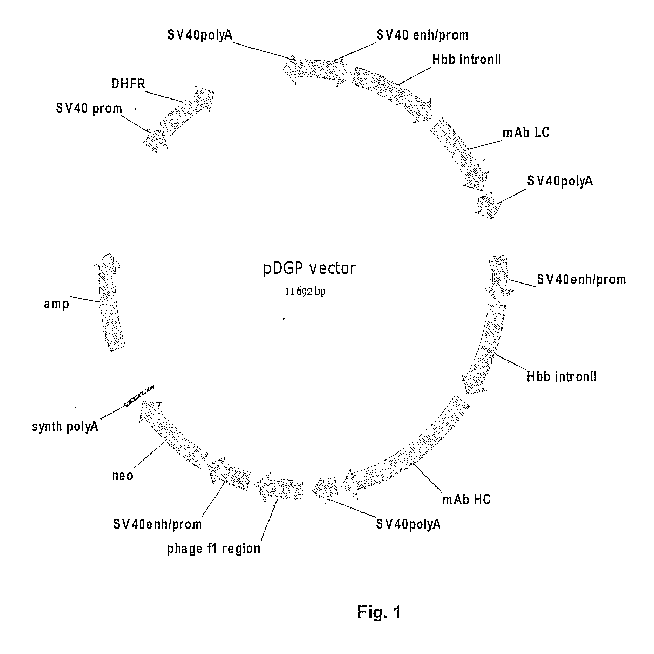

The present invention describes new mammalian expression vectors (with or without gene(s) of interest, GOI) for integration into the mammalian genome, the vectors comprising a novel combination of regulatory elements. In particular, the present invention describes expression vectors comprising the following regulatory elements: the simian virus 40 enhancer and early promoter region and the Hbb intronII, or fragments, derivatives and modifications thereof, wherein the expression vector is capable to take up at least one gene of interest and enable its/their expression. Preferably, such expression vector comprises at least one selection marker gene (e.g., pDGP including gene(s) of interest, see Fig. 1; e.g., pDGPΔGOI without gene(s) of interest). The vector (without gene(s) of interest) allows for incorporation of at least one, preferably two or more genes of interest, its/their subsequent expression, and for selection of transfected cells using, e.g., geneticin (G418) and/or methotrexate (MTX). The pDGPΔGOI vector as an example for a mammalian expression vector according to the present invention exhibits a 9555 bp sequence, one strand of which is represented by SEQ ID NO:2.

Currently many successive selection steps on a large number of cell pools have to be performed to generate and screen for a high producing cell pool before cell cloning. As a result, cell line development is a time-consuming and work-intensive activity.

There is a vast number of publications addressing the influence of regulatory elements on expression levels.

The positive effect of different intron sequences positioned in or just before the GOI coding sequence has been known for a long time and described in many publications (2, 4, 5, 6). Studies addressing transcriptional efficiency of different promoters were conducted identifying the CMV promoter as the most efficient (2, 3, 4, 5, 6). For this reason the GOI expression cassettes in most mammalian expression vectors today are driven by a CMV promoter and have an intron sequence just upstream of the GOI coding sequence.

Concerning the combination of GOI expression cassettes and selection marker genes in a particular expression vector, several combinations were reported and functionally tested (1). For monoclonal antibody (mAb) expression, solutions with two vectors expressing separately the light chain and heavy chain were described as well as vectors harbouring expression cassettes for both mAb chains together with the *neo* and *dhfr* expression cassettes on the same plasmid (1).

In most cases only transient expression levels using intracellular reporter proteins like luciferase (2, 5, 6) were determined. By this approach it is hard to predict how a regulatory element and/or vector after integration into a chromosome will influence stable expression levels of a secreted protein, where the final expression level is the result of a complex interplay between transcription, translation, intracellular trafficking, and secretion rates. Although most important for the biopharmaceutical industry, this question is rarely addressed in the literature (3). In particular, it would seem that the relevance of a specific combination of regulatory elements and selection markers for the time needed to develop a cell line and the efficiency of cell line development has never been addressed. Consequently, the principal object of the present invention was to look for a combination of regulatory elements and selection markers superior to those known in the art in terms of the above mentioned parameters time and efficiency.

Accordingly, the present invention overcomes the downside described above and provides mammalian expression vectors for integration into the respective mammalian genome (in particular the vector, if devoid of gene(s) of interest, designated pDGPΔGOI exhibiting the sequence of SEQ ID NO:2; and the vector designated pDGP, if including the gene(s) of interest, see Fig. 1) comprising a novel combination of regulatory elements: the simian virus 40 (SV40) enhancer and early promoter region, Hbb intronII, or fragments, derivatives and modifications of that intron (which is distinct from and hence must not be confused with the chimeric intron described in the literature, e.g., Xu et al. (2)), and optionally at least one selection marker gene (e.g., *neo, dhfr*). Such expression vectors are suitable to take up at least one gene of interest and, if so, enable its/their expression and subsequent selection of transfected cells using at least one suitable selection agent (e.g., G418 and/or MTX). Even one step-selection with G418 selects for cells expressing the at least one polypeptide encoded by the at least one gene of interest in high amounts and in a highly reproducible manner, thereby reducing the time to develop cell lines and work load significantly.

As a side note, the following should be taken into account: Expression vectors integrating into the genome that are known in the prior art upon two or more selection steps (one of which is usually MTX selection) regularly generate cells that harbour several copies of the GOI integrated into their genome and therefore express the GOI at an increased rate. Quite conversely, the expression vectors of the present invention do not require multiple copies thereof to be integrated into the genome and/or two or more rounds of selection steps in order to allow the mammalian host cell to express the GOI at an increased rate. This is the immediate outcome of the combination of the regulatory elements according to the present invention and resides in increased expression rates irrespective of the number of gene copies integrated into the genome.

The advantage of the present invention is thus that there is no need to perform at least one selection step each with G418 and MTX in order to accomplish satisfactory expression rates but that only one selection step either with G418 or MTX, to mention exemplary selection systems, is sufficient.

The term "Hbb intronII" as used herein relates to any nucleic acid molecule exhibiting a sequence of the second intron of the human β-globin (*hbb*) gene (Hbb intron 2) including 10 bp of the sequence immediately upstream of said Hbb intron 2 in the *hbb* gene and 10 bp of the sequence immediately downstream of the Hbb intron 2 in the *hbb* gene. According to the present invention, the term "fragments, derivatives, and modifications of Hbb intronII" encompasses nucleic acid molecules which are capable of hybridising to Hbb intronII under stringent hybridisation conditions and have the same expression enhancing activity (when combined with the SV 40 enhancer and early promoter region) as has the Hbb intronII in that SV 40 enhancer/promoter combination. In particular, the term "fragments, derivatives, and modifications of Hbb intronII" encompasses nucleic acid molecules which hybridise to the strand of a nucleic acid molecule exhibiting the sequence of SEQ ID NO:1, or to its complement, or parts thereof, under stringent hybridisation conditions and which have the same expression enhancing activity as the nucleic acid molecule exhibiting the sequence of SEQ ID NO:1 in the same SV 40 enhancer/promoter combination. According to a preferred embodiment of the present invention, the Hbb intronII exhibits the sequence set out in SEQ ID NO:1.

It is well known in the art how to perform hybridisation experiments with nucleic acid molecules, i.e., the person skilled in the art knows what hybridisation conditions s/he has to use in accordance with the present invention. Such hybridisation conditions are referred to in standard text books such as Molecular Cloning A Laboratory Manual (Sambrook J., Russell D.W.) Cold Spring Harbor Laboratory (2001) N.Y., Current Protocols in Molecular Biology (eds.: Ausubel F.M., Brent R., Kingston R.E., Moore D.D., Seidman J.G, Smith J.A., Struhl K.) [Core publication in 1987 - quarterly updated] Copyright © 2007 by John Wiley and Sons, Inc..

"Stringent hybridisation conditions" refer, e.g., to an overnight incubation at 40-60°C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 mg/ml denatured, sheared salmon sperm DNA, followed by washing the filters in 0.1x SSC at about 65°C. Also contemplated are nucleic acid molecules that hybridise to the nucleic acid molecule exhibiting the sequence of SEQ ID NO:1, or to its complement, or parts thereof, at lower stringency hybridisation conditions. Changes in the stringency of hybridisation and signal detection are primarily accomplished through the manipulation of formamide concentration (lower percentages of formamide result in lowered stringency); salt conditions, or temperature. For example, lower stringency conditions include an overnight incubation at 37°C in a solution comprising 6X SSPE (20X SSPE = 3M NaCl; 0.2M NaH₂PO₄; 0.02M EDTA, pH 7.4), 0.5% SDS, 30% formamide, 100 mg/ml denatured, sheared salmon sperm DNA; followed by washes at 50°C with 1X SSPE, 0.1% SDS. In addition, to achieve even lower stringency, washes performed following stringent hybridisation can be done at higher salt concentrations (e.g., 5X SSC). It is of note that variations in the above conditions may be accomplished through the inclusion and/or substitution of alternate blocking reagents used to suppress background in hybridisation experiments. Typical blocking reagents include Denhardt's reagent, BLOTTO, heparin, denatured salmon sperm DNA, and commercially available proprietary formulation. The inclusion of specific blocking reagents may require modification of the hybridisation conditions described above, due to problems with compatibility.

Thus, a first aspect of the present invention includes the provision of an expression vector for integration into the mammalian genome, said expression vector comprising the following regulatory elements: the simian virus 40 (SV40) enhancer and early promoter region and the Hbb intronII, or fragments, derivatives and modifications thereof, and preferably the intron exhibiting the sequence of SEQ ID NO:1, wherein the expression vector is capable to take up at least one gene of interest and enable its/their expression.

Preferred embodiments of that aspect of the present invention are
(i) a vector of the first aspect of the present invention, wherein the vector comprises at least one gene encoding a eukaryotic and/or prokaryotic selection marker, in particular the neomycin phosphotransferase gene (*neo*) from Tn5 and/or the dihydrofolate reductase (*dhfr*) gene;
(ii) a vector of the first aspect of the present invention, wherein the vector is circular;
(iii) a vector of the first aspect of the present invention, wherein the vector comprises at least one gene of interest under the control of said regulatory elements;
(iv) a vector of the first aspect of the present invention, wherein the at least one gene of interest is the gene encoding the light chain of an immunoglobulin and/or the gene encoding the heavy chain of an immunoglobulin;
(v) a vector of the first aspect of the present invention, wherein the order of the regulatory elements, the genes of interest, and the genes encoding a selection marker is as follows: 5'-SV40 enhancer/early promoter region, Hbb intronII, or fragments, derivatives and modifications thereof, gene encoding the light chain of an immunoglobulin, SV40 polyadenylation signal sequence; SV40 enhancer/early promoter region, Hbb intronII, or fragments, derivatives and modifications thereof, gene encoding the heavy chain of an immunoglobulin, SV40 polyadenylation signal sequence; SV40 enhancer/early promoter region, Tn5 neomycin phosphotransferase gene, a synthetic polyadenylation signal sequence, SV40 early promoter without enhancer sequence, dihydrofolate reductase gene, SV40 polyadenylation signal sequence-3';
(vi) a vector of the first aspect of the present invention, wherein the vector comprises at least one prokaryotic selection marker gene, e.g., an antibiotic resistance gene such as the ampicillin resistance gene, and/or further regulatory elements known by the skilled artisan to be advantageously included into a mammalian expression vector; and
(vii) a vector of the first aspect of the present invention, wherein the vector is the pDGPΔGOI vector exhibiting a 9555 bp sequence, one strand of which is represented by the sequence of SEQ ID NO:2.

A second aspect of the present invention is a method for producing an expression vector of the first aspect of the present invention, wherein said method comprises the step of arranging the following regulatory genetic elements (a) and (b) in a vector comprising further elements commonly required for the expression vector to be functional and to integrate into the mammalian host genome: (a) the simian vitus 40 enhancer and early promoter region and (b) the Hbb intronII, or fragments, derivatives and modifications thereof.

A preferred embodiment of that second aspect of the present invention is a method further comprising the step of arranging (c) at least one gene of interest such as to enable the vector to confer its/their expression and/or (d) at least one selection marker gene. According to another preferred embodiment, the at least one selection marker gene is the neomycin phosphotransferase gene from Tn5 and/or the dihydrofolate reductase gene.

A third aspect of the present invention is a vector system comprising at least two vectors as defined in any of preferred embodiments (i), (ii), and (iii), wherein each of the at least two vectors comprises at least one gene of interest. Preferred is such vector system, wherein a first gene of interest located on a first vector encodes the light chain of an immunoglobulin, and a second gene of interest located on a second vector encodes the heavy chain of an immunoglobulin. Another vector system also contemplated by the present invention comprises at least two vectors, wherein a first vector is according to the first aspect of the present invention but having no eukaryotic selection marker, and wherein a second vector comprises at least one gene encoding a selection marker conferring resistance to a transfected cell.

A fourth aspect of the present invention is a mammalian cell comprising an expression vector of any of preferred embodiments (i), (ii), (iii), (iv), (v), (vi), and (vii) of the first aspect of the present invention or a vector system according to the third aspect of the present invention. Basically any mammalian cell can be used in conjunction with the present invention as long as it allows the recombinant expression of a polypeptide.

According to a preferred embodiment of that fourth aspect of the present invention such cell is a COP cell, an L cell, a C127 cell, an Sp2/0 cell, an NS-0 cell, an NS-1 cell, an NIH3T3 cell, a PC12 cell, a PC12h cell, a BHK cell, a CHO cell, a COS1 cell, a COS3 cell, a COS7 cell, a CV1 cell, a Vero cell, a HeLa cell, an HEK-293 cell, a PER C6 cell, or a cell derived from diploid fibroblasts, myeloma cells, and HepG2.

A fifth aspect of the present invention relates to the method for producing a cell of the fourth aspect of the present invention, the method comprising the step of providing, and preferably transfecting, a mammalian cell with an expression vector of any of preferred embodiments (i), (ii), (iii), (iv), (v), (vi), and (vii) of the first aspect of the present invention, or with a vector system according to the third aspect of the present invention.

A sixth aspect of the present invention is a method for producing at least one polypeptide of interest, wherein said method comprises the step of culturing a cell of the fourth aspect of the present invention in a cell culture medium under conditions allowing expression of said at least one polypeptide of interest.

According to a preferred embodiment of that sixth aspect of the present invention the at least one polypeptide of interest is produced by culturing a CHO cell according to the fourth aspect of the present invention. The vectors according to the present invention are particularly suitable for producing polypeptides in rodent cells such as CHO cells. According to another preferred embodiment of that sixth aspect of the present invention such method further comprises the step of isolating from the cell culture medium said at least one polypeptide of interest.

A seventh aspect of the present invention relates to the use of the Hob intronII, or of fragments, derivatives, and modifications thereof, but preferably of the Hbb intronII exhibiting the sequence of SEQ ID NO:1, the intro preferably being in combination with the SV 40 enhancer and early promoter region, in a mammalian expression vector for the enhancement of heterologous gene expression in mammalian expression systems.

According to a preferred embodiment of the seventh aspect of the present invention such use is defined by the Hbb intronII's, its fragment's, derivative's, and modification's location between the sequence encoding the heterologous gene and the SV 40 enhancer and early promoter region driving the expression of the heterologous gene.

Especially preferred embodiments of the invention include the following methods and uses:
A. A method for producing an expression vector of claim 1 or 2, wherein said method comprises the step of arranging regulatory elements (a) and (b) into a vector comprising further elements commonly required for a functional expression vector: (a) the simian virus 40 enhancer and early promoter region and (b) the Hbb intronII, or fragments, derivatives and modifications thereof.
B. The method of A, wherein the method further comprises the step of arranging (c) at least one gene of interest such as to enable the vector to confer its/their expression and/or (d) at least one gene encoding a selection marker.
C. The method of B, wherein the at least one selection marker gene is the neomycin phosphotransferase gene from Tn5 and/or the dihydrofolate reductase gene.
D. Use of the Hbb intronII, or of a fragment, a derivative, or a modification thereof, in combination with the simian virus 40 enhancer and early promoter region driving the expression of a heterologous gene in a mammalian expression vector for the enhancement of heterologous gene expression in mammalian expression systems.
E. Use of D, wherein the Hbb intronII exhibits the sequence of SEQ ID NO:1.
F. Use of D or E, wherein the Hbb intronII, or its fragment, derivative, or modification, is located between the sequence encoding the heterologous gene and the SV 40 enhancer and early promoter region.

The mammalian expression vectors of the present invention exhibiting two or more GOI expression cassettes are particularly useful for the expression of multimeric, in particular heteromultimeric proteins including monoclonal antibodies. Vectors of the present invention exhibiting only one GOI expression cassette can be advantageously used to express monomeric proteins such as cytokines and hormones. When multimeric proteins, in particular monoclonal antibodies, are to be expressed, a vector system is also contemplated consisting of at least two vectors of the present invention, wherein a first vector comprises a first gene of interest and a second vector comprises a second gene of interest. Preferably, in said vector system, said first gene of interest located on the first vector encodes the light chain of all immunoglobulin and the second gene of interest located on the second vector encodes the heavy chain of an immunoglobulin.

Genetic elements (see the abbreviations utilised in Fig. 1) comprised in the vectors according to the present invention (and in particular in pDGPΔGOI/pDGP) are:
- SV40 enh/prom: the simian virus 40 enhancer and early promoter region;
- Hbb intronII, or fragments, derivatives and modifications thereof, as defined above.

Genetic elements (see the abbreviations utilised in Fig. 1) that may be comprised in the vectors according to the present invention (and in particular in pDGPΔGOI/pDGP) are:
- *neo*: the aminoglycoside-3'-phosphotransferase (APH 3' II) gene, also named neomycin phosphotransferase gene, from Tn5; APH 3' II inactivates the antibiotic geneticin (G418);
- *dhfr*: the dihydrofolate reductase gene;
- SV40 polyA: the polyadenylation signal sequence from SV40;
- synth polyA: a synthetic polyadenylation signal based on the polyadenylation signal of the rabbit β-globin gene.

The pDGP vector as one embodiment exemplifying the present invention (for a vector with at least one gene of interest) is schematically depicted in Fig. 1. It contains two GOI expression cassettes (each exhibiting basically identical structures and regulatory elements, except that the genes to be expressed are distinct: the light and heavy chain gene, respectively, of a monoclonal antibody, the expression cassettes thus enabling expression of two distinct genes of interest in mammalian cells.

Genes of interest typically are cDNA sequences, but may likewise be (genomic) DNA sequences.

As mentioned previously, the vectors according to the present invention (and in particular pDGPΔGOI/pDGP) may also contain genes/expression cassettes encoding a selection marker. Preferred selection markers are the neomycin phosphotransferase encoded by the *neo* gene/expression cassette and the dihydrofolate reductase encoded by the *dhfr* gene/expression cassette. The genes/expression cassettes encoding the selectable markers allow for selection of transfected cells by, e.g., geneticin (G418) and methotrexate (MTX). The *neo* expression cassette of pDGP contains the neomycin phosphotransferase gene driven by the SV40 enhancer and early promoter region and is further transcriptionally regulated by a synthetic polyadenylation signal sequence (as to be seen in Fig. 1). The neomycin phosphotransferase gene originates from bacteria and is expressed in mammalian cells if under the control of the regulatory elements mentioned above. Quite conversely, the *dhfr* expression cassette of pDGP contains the *dhfr* gene driven by the SV40 early promoter region (without enhancer sequence) and is further transcriptionally regulated by the SV40 polyadenylation signal in 3'- to 5'-orientation. The enzyme dihydrofolate reductase (DHFR) is required for the conversion of dihydrofolate to tetrahydrofolate which, in turn, is required for nucleic acid synthesis in cells. Efficient cell selection is achieved by irreversible inhibition of dihydrofolate reductase by methotrexate. Thus, growth of mammalian cells successfully transfected with the pDGP vector (and thus overexpressing the DHFR protein) is enabled in a methotrexate-supplemented growth medium.

Figure 1 schematically depicts a vector in accordance with a preferred embodiment of the present invention (vector pDGP) including two genes of interest, that is, mAb HC and mAb LC (mAb: monoclonal antibody; HC: heavy chain; LC: light chain).

Figure 2 schematically depicts the control vector.

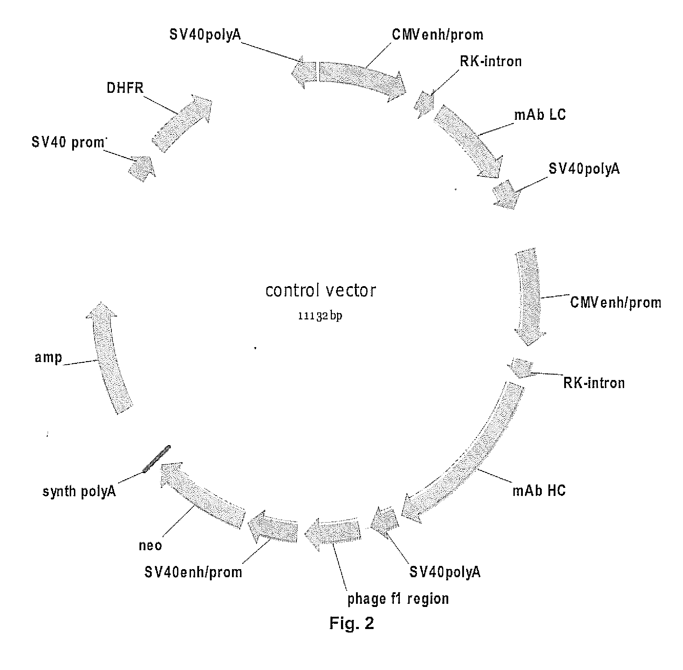

In an attempt to test the performance of the pDGP expression vector according to the present invention the inventors compared pDGP to the control vector (depicted in Fig. 2). Both the pDGP and the control vector include the same two genes of interest: mAb HC and mAb LC. Both vectors were transfected into the same host cell lines (e.g., CHO K1PD, COS1, CV1, Vero, HeLa, HEK-293, PER C6). Cells were then subjected to successive selection steps in the presence of G418 and MTX. After each selection step mAb titres in standard shake flask batches were determined and compared. Experimental details are presented below.

### Experiments

### Expression vector design and construction

Vectors used in this study were designed *in silico* using vector NTI 9.0 evaluation software. Fragments required for the construction of vectors such as the pDGP vector and the control vector (Fig. 2) were prepared at Geneart AG, Regensburg, Germany. It is understood by those of skill in the art that several vector assemblies according to the teachings of the present invention are feasible. As the individual elements of the vectors according to the invention are known in the art, suitable vectors can be assembled e.g. by sequencing or amplification and appropriate cloning of the basic genetic elements and expression cassettes. Hence, it will be understood that several other embodiments and ways to obtain respective vectors are suitable and readily available.

Regulatory genetic elements driving expression of the 4 expression cassettes in each expression vector are listed in Table 1.

**Table 1: List of regulatory genetic elements comprised in the pDGP/control vector**

| **Vector** | **GOI(2)** | | | ***neo*** | | ***dhfr*** | |
|---|---|---|---|---|---|---|---|
| | **promoter** | **intron** | **polyA** | **promoter** | **polyA** | **promoter** | **polyA** |
| **pDGP vector** | SV40 enh/prom | Hbb intronII | SV40 polyA | SV40 enh/prom | synth polyA | SV40 prom | SV40 polyA |
| **Control vector** | CMV enh/prom | RK-intron | SV40 polyA | SV40 enh/prom | synth polyA | SV40 prom | SV40 polyA |

### Vector preparation for transfection

Both expression vectors were linearised using single cutter restriction endonuclease *Swa*I (*ATTT AAAT*, Roche, Cat. No. 11 371 525 001). A maximum of 100 µg of vector DNA per reaction was digested using *Swa*I at 5U/µg DNA. Appropriate amounts of 10x reaction buffer and H₂O were added to the reaction mixtures. Reaction mixtures were incubated at 25°C 4 h-overnight. After digestion DNA precipitation was performed in a laminar air flow chamber as described below:
- Add 1 volume of isopropanol
- Vortex thoroughly
- Centrifuge 30 min at 21000 x g at 4°C
- Discard the supernatant
- Add carefully 1 volume of sterile, ice cold 70% ethanol
- Centrifuge 1 min at max. speed, at 4°C
- Discard the supernatant
- Air dry pellet at RT for 5-30 min (in laminar)
- Resuspend DNA in 100 µl sterile water.

After digestion the purity (OD 260/280) and concentration of linear DNA was determined using NanoDrop ND-1000. Gel electrophoresis of digested DNA (0.8% agarose e-gel, Invitrogen) was performed to control the linearization.

### Host cells

CHO K1PD, COS1, CV1, Vero, HeLa, HEK-293, PER C6 cells were used.

The CHO K1PD cell line is a subpopulation of the CHO K1 cell line originating from ATCC (Cat. No. CCL-61.3). The original cell line was adapted to serum-free suspension culture in DM122 medium. DM122 growth medium was used for all experiments employing CHO K1PD cells.

### Transfection

The Amaxa system was used for cell transfection (Nucleofector kit V, Cat. No.: VCA-1003). No more than 5 pools are transfected at once, to enable sufficient time for all necessary cell manipulations. The detailed protocol is as follows:
1. At the time of transfection, cells should be up to 2 x 10⁶/ml with a viability ≥ 90%.
2. 5 x 10⁶ cells are used per transfection.
3. Count cells and centrifuge at 90 x g, 10 min, RT in a 50 ml centrifuge tube.
4. Carefully remove the medium and resuspend the cell pellet in 100 µl of solution V (contained in the Amaxa kit)
5. Add DNA (3 µg) and mix gently.
6. Add the mixture from step 5 to a transfection cuvette and place same in an Amaxa Nucleofector device.
7. Transfect the cells using Amaxa program U 23.
8. For cells growing in shake flasks (e.g., CHO K1PD) add some growth medium in the cuvette and transfer the cells carefully to a 125 ml shake flask by utilising 20 ml of the growth medium. Rinse the cuvette once or twice with fresh growth medium and add it to the shake flask. Incubate the cells 24 - 48 h in a shaker (110 rpm), at 37°C, 10% CO₂.

### Growth medium and G418/MTX selection

The cells (e.g. CHO K1PD, COS1, CV1, Vero, HeLa, HEK-293, PER C6 cells) were cultivated in a suitable medium for culturing mammalian cells, such as DM122 growth medium supplemented with 8 mM L-glutamine (Sigma, Cat. No. G7513) when using CHO K1PD for the experiments.
Selection steps were performed in the same medium additionally supplemented with G418 (Geneticin, Gibco, Cat. No. 10131-027; final concentration: 0.8 mg/ml) and/or methotrexate (methotrexate hydrate, Sigma, Cat. No. M8407150; final concentration: 150 or 250 nM).
G418 selection was the first selection step after transfection. G418 was added to the cells 2-5 days after transfection when cell viability exceeded 60%. G418 selection usually takes 2-4 weeks. Once cell viability had reached at least 85%, MTX selection was carried out.

A seeding density of 2 x 10⁵ viable cells per ml was used to start MTX selection on G418 preselected cells. Two selection sub-steps were performed using the above indicated MTX concentrations (150/250 nM). MTX selection usually takes 2 to 3 weeks, until cell viability reached more than 85%.

### Thawing/freezing of cells

The cells were thawed at 37°C and drop-wise inoculated directly to 250 ml shake flasks containing 50 ml pre-warmed medium at an initial cell density of about 10⁵ viable cells per ml. Cells were cultured at 37°C, 10% CO₂, 110 rpm.

Cells were frozen in exponential growth phase at viability >90 %. 5-10 x 10⁶ viable cells per vial were frozen in conditioned medium containing 7.5% DMSO. First, the cell culture was centrifuged at 180 x g, 5 min, RT. Second, the supernatant was partially removed. Then, DMSO was added to the remaining supernatant to a final concentration of 7.5%. Cell pellets were gently resuspended. Cryo-vials were filled with 1 ml of the cell suspension each and transferred into a - 80°C deep freezer in a Mr. Frosty cryo box. Within 1 month the frozen cells in the cryo-vials were transferred into a liquid nitrogen container.

### Culture and handling of cells

Cells were split every 2-3 days at a density of 1-1.5 x 10⁵ viable cells per ml and added to the appropriate pre-warmed growth medium to maintain exponential growth.
Incubation conditions: 37°C, 90-110 rpm (for 125 & 250 ml shake flasks), 10% CO₂ (for DM122 medium).

### Testing for mAb expression

After each selection step (G418, MTX) a ten-day batch experiment in appropriately supplemented medium (e.g., DM122) was performed to evaluate the expression of mAb. Cells were seeded at a final density of 1.5 x 10⁵ viable cells/ml in 125 ml shake flasks. The total culture volumes were 25 ml. Cells were cultivated in a shaking incubator (37°C, 110 rpm, 10% CO₂). At day 10, 1 ml cell culture supernatant samples were taken to measure the mAb titre. The mAb content was determined by AC (Affinity Chromatography with Protein A).

### Results

Ten-day batch titres of cells transfected with pDGP vector after different selection steps are shown in Table 2.

**Table 2: Ten-day batch titres; cells transfected with pDGP vector after different selection steps**

| **Experiment** | **G418** | **150 nM MTX** | **250 nM MTX** |
|---|---|---|---|
| | **Titre (mg/l)** | **Titre (mg/l)** | **Titre (mg/l)** |
| 1 | 94 | 246 | 281 |
| 2 | 80 | 249 | 284 |
| 3 | 94 | 225 | 288 |
| 4 | 96 | 229 | 303 |
| 5 | 91 | 218 | 309 |
| 6 | 84 | | |
| 7 | 74 | 212 | 339 |
| 8 | 89 | 216 | 289 |
| 9 | 89 | | |
| 10 | 94 | 243 | 260 |

Ten-day batch titres of cells transfected with control vector after different selection steps are shown in Table 3

**Table 3: Ten-day batch titres; cells transfected with control vector after different selection steps**

| **Experiment** | **G418** | **150 nM MTX** | **250 nM MTX** |
|---|---|---|---|
| | **Titre (mg/l)** | **Titre (mg/l)** | **Titre (mg/l)** |
| 1 | 15 | 190 | 192 |
| 2 | 15 | 247 | 372 |
| 3 | 14 | 251 | 219 |
| 4 | 15 | 207 | 341 |
| 5 | 15 | 154 | 385 |
| 6 | 15 | 422 | 646 |

Compared to the cells transfected with the control vector, 4-5 times higher titres were measured for cells transfected with pDGP after G418 selection. In average, the same titres were measured for both experimental approaches after MTX selection. However, the achieved titres were much more reproducible with the cells transfected with pDGP (±10%) compared to the cells transfected with control vector (±50%).

### Conclusion

The expression vectors of the present invention (such as pDGP) provide the following advantages over expression vectors currently used in the art (such as the control vector):
- Due to high reproducibility, the use of pDGP, as an example of the vectors according to the invention, considerably reduces the number of cell samples (i.e., transfected cell pools) to be generated and tested to only 3 to 5, thereby reducing the work load significantly. Currently, the generation of a high number of cell samples (up to 50) due to low reproducibility is generally required to identify high producing cell samples.
- The use of pDGP provides adequate titres of the expressed protein of interest for product characterisation, preclinical and phase I studies after just one single G418 selection step. Additional MTX selection steps to increase the protein (e.g., antibody) titre prior to cell cloning are not required.
- The time required to develop a cell line can be remarkably reduced when using pDGP, as the number of selection steps is reduced to one G418 step only.

### References

1 Trill JJ, Shatzman AR, Ganguly S: Production of monoclonal antibodies in COS and CHO cells. Current Opinion in Biotechnology 6, 1995: 553-560
2 Xu Z, Mizuguchi H, Ishii-Watabe A, Uchida E, Mayumi T, Hayakawa T: Optimization of transcriptional regulatory elements for constructing plasmid vectors. Gene 272, 2006: 149-156
3 Melcher R, Grosch HW, Hasilik A: Plasmid vectors with a 5'-hybrid intron facilitate high-level glycoprotein expression in CHO-cells. Biochimica et Biophysica Acta, 1575, 2002: 49-53
4 Kim Y, Kim S, Lee J, Shin, H: Expression vector for mammal comprising murine cytomegalovirus immediate early gene enhancer/promoter and human beta globin intron, cell transformed therewith and method for producing heterologous protein using said cell. Patent: KR 1020000070503-A2 24-NOV-2000
5 Xu Z, Mizuguchi H, Ishii-Watabe A, Uchida E, Mayumi T, Hayakawa T: Strength evaluation of transcriptional regulatory elements for transgene expression by adenovirus vector. Journal of Controlled Release, 81, 2002: 155-163
6 Komiya E, Kondoh M, Mizuguchi H, Fujii M, Utoguchi N, Nakanishi T, Watanabe Y: Characteristics of Transcription-regulatory Elements for Gene Expression from Plasmid Vectors in Human Trophoblast Cell Lines. Placenta, 27, 2006: 934-938

## Claims

1. A mammalian expression vector comprising the following regulatory elements:
(a) the simian virus 40 enhancer and early promoter region and
(b) the Hbb intronII, or fragments, derivatives and modifications thereof,
wherein the expression vector is capable to take up at least one gene of interest and enable its/their expression.

2. The vector of claim 1, wherein the Hbb intronII exhibits the sequence of SEQ ID NO:1.

3. The vector of claims 1 or 2, wherein the vector further comprises at least one gene encoding a eukaryotic and/or prokaryotic selection marker, in particular wherein the at least one eukaryotic selection marker gene is the neomycin phosphotransferase gene from Tn5 and/or the dihydrofolate reductase gene.

4. The vector of any of the preceding claims, wherein the vector is circular and/or comprises at least one gene of interest under the control of the regulatory elements defined in claim 1 (a) and (b).

5. The vector of claim 4, wherein the vector comprises at least one gene of interest and wherein said at least one gene encodes the light chain of an immunoglobulin and/or the heavy chain of an immunoglobulin.

6. The vector of any of claims 1 to 5, wherein the order of the regulatory elements, the gene(s) of interest, and the genes encoding a selection marker is as follows: 5'-SV40 enhancer/early promoter region, Hbb intronII, or fragments, derivatives and modifications thereof, gene encoding the light chain of an immunoglobulin, SV40 polyadenylation signal sequence; SV40 enhancer/early promoter region, Hbb intronII, or fragments, derivatives and modifications thereof, gene encoding the heavy chain of an immunoglobulin, SV40 polyadenylation signal sequence; SV40 enhancer/early promoter region, Tn5 neomycin phosphotransferase gene, a synthetic polyadenylation signal sequence; SV40 early promoter, dihydrofolate reductase gene, SV40 polyadenylation signal sequence-3'.

7. The vector of any of claims 1 to 6, wherein the vector is the pDGPΔGOI vector exhibiting a 9555 bp sequence, one strand of which being represented by SEQ ID NO:2.

8. A vector system, comprising at least two vectors as defined in claim 3 or 4, wherein each of the at least two vectors comprises at least one gene of interest.

9. The vector system of claim 8, wherein a first gene of interest located on a first vector encodes the light chain of an immunoglobulin, and a second gene of interest located on a second vector encodes the heavy chain of an immunoglobulin.

10. A vector system, comprising at least two vectors, wherein a first vector is defined as in claim 1 or 2 and wherein a second vector comprises at least one gene encoding a selection marker conferring resistance to a transfected cell.

11. A mammalian cell comprising an expression vector of any of claims 3 to 7, or comprising a vector system of any of claims 8 to 10.

12. The cell of claim 11, wherein the cell is a COP cell, an L cell, a C127 cell, an Sp2/0 cell, an NS-0 cell, an NS-1 cell, an NIH3T3 cell, a PC12 cell, a PC12h cell, a BHK cell, a CHO cell, a COS1 cell, a COS3 cell, a COS7 cell, a CV1 cell, a Vero cell, a HeLa cell, an HEK-293 cell, a PER C6 cell, or a cell derived from diploid fibroblasts, myeloma cells, and HepG2.

13. A method for producing a cell of claim 11, the method comprising the step of providing a mammalian cell with an expression vector of any of claims 3 to 7, or with a vector system of any of claims 8 to 10.

14. A method for producing at least one polypeptide of interest, wherein said method comprises the step of culturing a cell of claim 11 in a cell culture medium under conditions allowing expression of said at least one polypeptide of interest.

15. The method of claim 14, wherein said at least one polypeptide of interest is/are secreted into the cell culture medium and said method further comprises the step of isolating from the cell culture medium said at least one polypeptide of interest.


SEQUENCE LISTING

<110>  Lek Pharmaceuticals d.d.

<120>  Mammalian expression vector

<130>  HEX-001 EP 1

<160>  2

<170>  PatentIn version 3.3

<210>  1
<211>  870
<212>  DNA
<213>  Artificial Sequence

<220>
<223>  Synthetic Sequence

<400>  1
   gaacttcagg gtgagtctat gggacccttg atgttttctt tccccttctt ttctatggtt      60
   aagttcatgt cataggaagg ggagaagtaa cagggtacag tttagaatgg gaaacagacg     120
   aatgattgca tcagtgtgga agtctcagga tcgttttagt ttcttttatt tgctgttcat     180
   aacaattgtt ttcttttgtt taattcttgc tttctttttt tttcttctcc gcaattttta     240
   ctattatact taatgcctta acattgtgta taacaaaagg aaatatctct gagatacatt     300
   aagtaactta aaaaaaaact ttacacagtc tgcctagtac attactattt ggaatatatg     360
   tgtgcttatt tgcatattca taatctccct actttatttt cttttatttt taattgatac     420
   ataatcatta tacatattta tgggttaaag tgtaatgttt taatatgtgt acacatattg     480
   accaaatcag ggtaattttg catttgtaat tttaaaaaat gctttcttct tttaatatac     540
   tttttgtttt atcttatttc taatactttc cctaatctct ttctttcagg gcaataatga     600
   tacaatgtat catgcctctt tgcaccattc taaagaataa cagtgataat ttctgggtta     660
   aggcaatagc aatatttctg catataaata tttctgcata taaattgtaa ctgatgtaag     720
   aggtttcata ttgctaatag cagctacaat ccagctacca ttctgctttt attttatggt     780
   tgggataagg ctggattatt ctgagtccaa gctaggccct tttgctaatc atgttcatac     840
   ctcttatctt cctcccacag ctcctgggca                                      870

<210>  2
<211>  9555
<212>  DNA
<213>  Artificial Sequence

<220>
<223>  Synthetic Sequence

<400>  2
aattcggatc tgcgcagcac catggcctga aataacctct gaaagaggaa cttggttagg      60

taccttctga ggcggaaaga accagctgtg gaatgtgtgt cagttagggt gtggaaagtc     120

cccaggctcc ccagcaggca gaagtatgca aagcatgcat ctcaattagt cagcaaccag 180

gtgtggaaag tccccaggct ccccagcagg cagaagtatg caaagcatgc atctcaatta 240

gtcagcaacc atagtcccgc ccctaactcc gcccatcccg cccctaactc cgcccagttc 300

cgcccattct ccgccccatg gctgactaat tttttttatt tatgcagagg ccgaggccgc 360

ctcggcctct gagctattcc agaagtagtg aggaggcttt tttggaggcc taggcttttg 420

caaaaagctt ctcgaggaac ttcagggtga gtctatggga cccttgatgt tttctttccc 480

cttcttttct atggttaagt tcatgtcata ggaaggggag aagtaacagg gtacagttta 540

gaatgggaaa cagacgaatg attgcatcag tgtggaagtc tcaggatcgt tttagtttct 600

tttatttgct gttcataaca attgttttct tttgtttaat tcttgctttc tttttttttc 660

ttctccgcaa tttttactat tatacttaat gccttaacat tgtgtataac aaaaggaaat 720

atctctgaga tacattaagt aacttaaaaa aaaactttac acagtctgcc tagtacatta 780

ctatttggaa tatatgtgtg cttatttgca tattcataat ctccctactt tattttcttt 840

tatttttaat tgatacataa tcattataca tatttatggg ttaaagtgta atgttttaat 900

atgtgtacac atattgacca aatcagggta attttgcatt tgtaatttta aaaaatgctt 960

tcttctttta atatactttt ttgtttatct tatttctaat actttcccta atctctttct 1020

ttcagggcaa taatgataca atgtatcatg cctctttgca ccattctaaa gaataacagt 1080

gataatttct gggttaaggc aatagcaata tttctgcata taaatatttc tgcatataaa 1140

ttgtaactga tgtaagaggt ttcatattgc taatagcagc tacaatccag ctaccattct 1200

gcttttattt tatggttggg ataaggctgg attattctga gtccaagcta ggcccttttg 1260

ctaatcatgt tcatacctct tatcttcctc ccacagctcc tgggcagtgt ccactcccag 1320

gtccaactgc acctcggttc tatcgaaaac gcgtgtcgac ccgggcggcc gcttcccttt 1380

agtgagtcga cccgggcggc cgcttccctt tagtgagggt taatgcttcg agcagacatg 1440

ataagataca ttgatgagtt tggacaaacc acaactagaa tgcagtgaaa aaaatgcttt 1500

atttgtgaaa tttgtgatgc tattgcttta tttgtaacca ttataagctg caataaacaa 1560

gttaacaaca acaattgcat tcattttatg tttcaggttc agggggagat gtgggaggtt 1620

ttttaaagca agtaaaacct ctacaaatgt ggtaaaatcc gataaggatc gatccgggct 1680

ggcgtaatag cgaagaggcc cgcaccgatc gcccttccca acagttgcgc agcctgaatg 1740

gcgaatggac gcgccctgta gcggcgcatt aagcgcggcg ggtgtggtgg ttacgcgcag 1800

cgtgaccgct acacttgcca gcgccctagc gcccgctcct ttcgctttct tcccttcctt 1860

tctcgccacg ttcgccggct ttccccgtca agctctaaat cgggggctcc ctttagggtt 1920

ccgatttaga gctttacggc acctcgaccg caaaaaactt gatttgggtg atggttcacg 1980

atctgcgcag caccatggcc tgaaataacc tctgaaagag gaacttggtt aggtaccttc 2040

tgaggcggaa agaaccagct gtggaatgtg tgtcagttag ggtgtggaaa gtccccaggc 2100

tccccagcag gcagaagtat gcaaagcatg catctcaatt agtcagcaac caggtgtgga 2160

aagtccccag gctccccagc aggcagaagt atgcaaagca tgcatctcaa ttagtcagca 2220

accatagtcc cgcccctaac tccgcccatc ccgcccctaa ctccgcccag ttccgcccat 2280

tctccgcccc atggctgact aatttttttt atttatgcag aggccgaggc cgcctcggcc 2340

tctgagctat tccagaagta gtgaggaggc ttttttggag gcctaggctt ttgcaaaaag 2400

cttctcgagg aacttcaggg tgagtctatg ggacccttga tgttttcttt ccccttcttt 2460

tctatggtta agttcatgtc ataggaaggg gagaagtaac agggtacagt ttagaatggg 2520

aaacagacga atgattgcat cagtgtggaa gtctcaggat cgttttagtt tcttttattt 2580

gctgttcata acaattgttt tcttttgttt aattcttgct ttcttttttt ttcttctccg 2640

caatttttac tattatactt aatgccttaa cattgtgtat aacaaaagga aatatctctg 2700

agatacatta agtaacttaa aaaaaaactt tacacagtct gcctagtaca ttactatttg 2760

gaatatatgt gtgcttattt gcatattcat aatctcccta ctttattttc ttttattttt 2820

aattgataca taatcattat acatatttat gggttaaagt gtaatgtttt aatatgtgta 2880

cacatattga ccaaatcagg gtaattttgc atttgtaatt ttaaaaaatg ctttcttctt 2940

ttaatatact tttttgttta tcttatttct aatactttcc ctaatctctt tctttcaggg 3000

caataatgat acaatgtatc atgcctcttt gcaccattct aaagaataac agtgataatt 3060

tctgggttaa ggcaatagca atatttctgc atataaatat ttctgcatat aaattgtaac 3120

tgatgtaaga ggtttcatat tgctaatagc agctacaatc cagctaccat tctgctttta 3180

ttttatggtt gggataaggc tggattattc tgagtccaag ctaggccctt ttgctaatca 3240

tgttcatacc tcttatcttc ctcccacagc tcctgggcag tgtccactcc caggtccaac 3300

tgcacctcgg ttctatcgaa aggcgcgtac tagtcatatg ggcgcgccgg gcggccgctt 3360

ccctttagtg agggttaatg cttcgagcag acatgataag atacattgat gagtttggac 3420

aaaccacaac tagaatgcag tgaaaaaaat gctttatttg tgaaatttgt gatgctattg 3480

ctttatttgt aaccattata agctgcaata aacaagttaa caacaacaat tgcattcatt 3540

ttatgtttca ggttcagggg gagatgtggg aggtttttta aagcaagtaa aacctctaca 3600

aatgtggtaa aatccgataa ggatcgatcc gggctggcgt aatagcgaag aggcccgcac 3660

cgatcgccct tcccaacagt tgcgcagcct gaatggcgaa tggacgcgcc ctgtagcggc 3720

gcattaagcg cggcgggtgt ggtggttacg cgcagcgtga ccgctacact tgccagcgcc 3780

ctagcgcccg ctcctttcgc tttcttccct tcctttctcg ccacgttcgc cggctttccc 3840

cgtcaagctc taaatcgggg gctcccttta gggttccgat ttagagcttt acggcacctc 3900

gaccgcaaaa aacttgattt gggtgatggt tcacgtagtg ggccatcgcc ctgatagacg 3960

gtttttcgcc ctttgacgtt ggagtccacg ttctttaata gtggactctt gttccaaact 4020

ggaacaacac tcaaccctat ctcggtctat tcttttgatt tataagggat tttgccgatt 4080

tcggcctatt ggttaaaaaa tgagctgatt taacaaatat ttaacgcgaa ttttaacaaa 4140

atattaacgt ttacaatttc gcctgatgcg gtattttctc cttacgcatc tgtgcggtat 4200

ttcacaccgc atacgcggat ctgcgcagca ccatggcctg aaataacctc tgaaagagga 4260

acttggttag gtaccttctg aggcggaaag aaccagctgt ggaatgtgtg tcagttaggg 4320

tgtggaaagt ccccaggctc cccagcaggc agaagtatgc aaagcatgca tctcaattag 4380

tcagcaacca ggtgtggaaa gtccccaggc tccccagcag gcagaagtat gcaaagcatg 4440

catctcaatt agtcagcaac catagtcccg cccctaactc cgcccatccc gcccctaact 4500

ccgcccagtt ccgcccattc tccgccccat ggctgactaa ttttttttat ttatgcagag 4560

gccgaggccg cctcggcctc tgagctattc cagaagtagt gaggaggctt ttttggaggc 4620

ctaggctttt gcaaaaagct tgattcttct gacacaacag tctcgaactt aaggctagag 4680

ccaccatgat tgaacaagat ggattgcacg caggttctcc ggccgcttgg gtggagaggc 4740

tattcggcta tgactgggca caacagacaa tcggctgctc tgatgccgcc gtgttccggc 4800

tgtcagcgca ggggcgcccg gttctttttg tcaagaccga cctgtccggt gccctgaatg 4860

aactgcagga cgaggcagcg cggctatcgt ggctggccac gacgggcgtt ccttgcgcag 4920

ctgtgctcga cgttgtcact gaagcgggaa gggactggct gctattgggc gaagtgccgg 4980

ggcaggatct cctgtcatct caccttgctc ctgccgagaa agtatccatc atggctgatg 5040

caatgcggcg gctgcatacg cttgatccgg ctacctgccc attcgaccac caagcgaaac 5100

atcgcatcga gcgagcacgt actcggatgg aagccggtct tgtcgatcag gatgatctgg 5160

acgaagagca tcaggggctc gcgccagccg aactgttcgc caggctcaag gcgcgcatgc 5220

ccgacggcga ggatctcgtc gtgacccatg gcgatgcctg cttgccgaat atcatggtgg 5280

aaaatggccg cttttctgga ttcatcgact gtggccggct gggtgtggcg gaccgctatc 5340

aggacatagc gttggctacc cgtgatattg ctgaagagct tggcggcgaa tgggctgacc 5400

gcttcctcgt gctttacggt atcgccgctc ccgattcgca gcgcatcgcc ttctatcgcc 5460

ttcttgacga gttcttctga gcgggactct ggggttcgaa atgaccgacc aagcgacgcc 5520

caacctgcca tcacgatggc cgcaataaaa tatctttatt ttcattacat ctgtgtgttg 5580

gttttttgtg tgaatcgata gcgataagga tccgcgtatg gtgcactctc agtacaatct 5640

gctctgatgc cgcatagtta agccagcccc gacacccgcc aacacccgct gacgcgccct 5700

gacgggcttg tctgctcccg gcatccgctt acagacaagc tgtgaccgtc tccgggagct 5760

gcatgtgtca gaggttttca ccgtcatcac cgaaacgcgc gagacgaaag ggcctcgtga 5820

tacgcctatt tttataggtt aatgtcatga taataatggt ttcttagacg tcaggtggca 5880

cttttcgggg aaatgtgcgc ggaaccccta tttgtttatt tttctaaata cattcaaata 5940

tgtatccgct catgagacaa taaccctgat aaatgcttca ataatattga aaaaggaaga 6000

gtatgagtat tcaacatttc cgtgtcgccc ttattccctt ttttgcggca ttttgccttc 6060

ctgtttttgc tcacccagaa acgctggtga aagtaaaaga tgctgaagat cagttgggtg 6120

cacgagtggg ttacatcgaa ctggatctca acagcggtaa gatccttgag agttttcgcc 6180

ccgaagaacg ttttccaatg atgagcactt ttaaagttct gctatgtggc gcggtattat 6240

cccgtattga cgccgggcaa gagcaactcg gtcgccgcat acactattct cagaatgact 6300

tggttgagta ctcaccagtc acagaaaagc atcttacgga tggcatgaca gtaagagaat 6360

tatgcagtgc tgccataacc atgagtgata acactgcggc caacttactt ctgacaacga 6420

tcggaggacc gaaggagcta accgcttttt tgcacaacat gggggatcat gtaactcgcc 6480

ttgatcgttg ggaaccggag ctgaatgaag ccataccaaa cgacgagcgt gacaccacga 6540

tgcctgtagc aatggcaaca acgttgcgca aactattaac tggcgaacta cttactctag 6600

cttcccggca acaattaata gactggatgg aggcggataa agttgcagga ccacttctgc 6660

gctcggccct tccggctggc tggtttattg ctgataaatc tggagccggt gagcgtgggt 6720

ctcgcggtat cattgcagca ctggggccag atggtaagcc ctcccgtatc gtagttatct 6780

acacgacggg gagtcaggca actatggatg aacgaaatag acagatcgct gagataggtg 6840

cctcactgat taagcattgg taactgtcag accaagttta ctcatatata ctttagattg 6900

atttaaaact tcatttttaa tttaaaagga tctaggtgaa gatccttttt gataatctca 6960

tgaccaaaat cccttaacgt gagttttcgt tccactgagc gtcagacccc gtagaaaaga 7020

tcaaaggatc ttcttgagat cctttttttc tgcgcgtaat ctgctgcttg caaacaaaaa 7080

aaccaccgct accagcggtg gtttgtttgc cggatcaaga gctaccaact ctttttccga 7140

aggtaactgg cttcagcaga gcgcagatac caaatactgt ccttctagtg tagccgtagt 7200

taggccacca cttcaagaac tctgtagcac cgcctacata cctcgctctg ctaatcctgt 7260

taccagtggc tgctgccagt ggcgataagt cgtgtcttac cgggttggac tcaagacgat 7320

agttaccgga taaggcgcag cggtcgggct gaacgggggg ttcgtgcaca cagcccagct 7380

tggagcgaac gacctacacc gaactgagat acctacagcg tgagctatga gaaagcgcca 7440

cgcttcccga agggagaaag gcggacaggt atccggtaag cggcagggtc ggaacaggag 7500

agcgcacgag ggagcttcca gggggaaacg cctggtatct ttatagtcct gtcgggtttc 7560

gccacctctg acttgagcgt cgatttttgt gatgctcgtc aggggggcgg agcctatgga 7620

aaaacgccag caacgcggcc tttttacggt tcctggcctt ttgctggcct tttgctcaca 7680

tggctcgaca gatccattta aattttcacc gtcatcaccg aaacgcgcga ggcagctgtg 7740

gaatgtgtgt cagttagggt gtggaaagtc cccaggctcc ccagcaggca gaagtatgca 7800

aagcatgcat ctcaattagt cagcaaccag gtgtggaaag tccccaggct ccccagcagg 7860

cagaagtatg caaagcatgc atctcaatta gtcagcaacc atagtcccgc ccctaactcc 7920

gcccatcccg cccctaactc cgcccagttc cgcccattct ccgccccatg gctgactaat 7980

tttttttatt tatgcagagg ccgaggccgc ctcggcctct gagctattcc agaagtagtg 8040

aggaggcttt tttggaggcc taggcttttg caaaaagctt tatccccgct gccatcatgg 8100

ttcgaccatt gaactgcatc gtcgccgtgt cccaagatat ggggattggc aagaacggag 8160

acctaccctg gcctccgctc aggaacgagt tcaagtactt ccaaagaatg accacaacct 8220

cttcagtgga aggtaaacag aatctggtga ttatgggtag gaaaacctgg ttctccattc 8280

ctgagaagaa tcgaccttta aaggacagaa ttaatatagt tctcagtaga gaactcaaag 8340

aaccaccacg aggagctcat tttcttgcca aaagtttgga tgatgcctta agacttattg 8400

aacaaccgga attggcaagt aaagtagaca tggtttggat agtcggaggc agttctgttt 8460

accaggaagc catgaatcaa ccaggccacc tcagactctt tgtgacaagg atcatgcagg 8520

aatttgaaag tgacacgttt ttcccagaaa ttgatttggg gaaatataaa cttctcccag 8580

aatacccagg cgtcctctct gaggtccagg aggaaaaagg catcaagtat aagtttgaag 8640

tctacgagaa gaaagactaa caggaagatg ctttcaagtt ctctgctccc ctcctaaagc 8700

tatgcatttt tataagacca tgggactttt gctggcttta gatctttgtg aaggaacctt 8760

acttctgtgg tgtgacataa ttggacaaac tacctacaga gatttaaagc tctaaggtaa 8820

atataaaatt tttaagtgta taatgtgtta aactactgat tctaattgtt tgtgtatttt 8880

agattccaac ctatggaact gatgaatggg agcagtggtg gaatgccttt aatgaggaaa 8940

acctgttttg ctcagaagaa atgccatcta gtgatgatga ggctactgct gactctcaac 9000

attctactcc tccaaaaaag aagagaaagg tagaagaccc caaggacttt ccttcagaat 9060

tgctaagttt tttgagtcat gctgtgttta gtaatagaac tcttgcttgc tttgctattt 9120

acaccacaaa ggaaaaagct gcactgctat acaagaaaat tatggaaaaa tattctgtaa 9180

cctttataag taggcataac agttataatc ataacatact gttttttctt actccacaca 9240

ggcatagagt gtctgctatt aataactatg ctcaaaaatt gtgtaccttt agctttttaa 9300

tttgtaaagg ggttaataag gaatatttga tgtatagtgc cttgactaga gatcataatc 9360

agccatacca catttgtaga ggttttactt gctttaaaaa acctcccaca cctccccctg 9420

aacctgaaac ataaaatgaa tgcaattgtt gttgttaact tgtttattgc agcttataat 9480

ggttacaaat aaagcaatag catcacaaat ttcacaaata aagcattttt ttcactgcat 9540

tctagttgtg gtttg 9555